# EUROPEAN PATENT SPECIFICATION

(11) **EP 3 151 814 B1**
(45) Date of publication and mention of the grant of the patent: **14.08.2019**
(21) Application number: 15726200.7
(22) Date of filing: 05.06.2015
(51) Int. Cl.: A61K 9/12, A61K 8/04, A61K 31/19, A61Q 19/00, A61B 18/02, A61M 35/00, B65D 83/28

(54) **TREATMENT OF A SKIN LESION**
BEHANDLUNG EINER HAUTLÄSION
TRAITEMENT D'UNE LÉSION DE LA PEAU

(30) Priority: 05.06.2014 EP 14171405
(43) Date of publication of application: 12.04.2017
(73) Proprietor: Medical Brands Research B.V., 1019 HC Amsterdam (NL)
(72) Inventor: HENDRIKS, Maikel, 1019 HC Amsterdam (NL)
(74) Representative: EDP Patent Attorneys B.V.
(86) International application number: PCT/EP2015/062613
(87) International publication number: WO 2015/185743

(56) References cited:
- WO-A1-2007/139378
- WO-A2-2012/007584
- US-A1- 2010 168 726
- COURTNEY E. GARRY ET AL: "Treatment of Warts", THE NEW ENGLAND JOURNAL OF MEDICINE, vol. 351, no. 16, 14 October 2004 (2004-10-14), pages 1692-1693, XP002742343,
- DATABASE WPI Section Ch, Week 199946 Thomson Scientific, London, GB; Class B07, AN 1999-543916 XP002742344, OKAI K; TAKEMURA T: "Spray for easy application of drug in freeze treatment of e.g. warts, corns - comprises jar containing coolant sprayed through injection nozzle connected to jet nozzle", -& JP 11 228388 A (NICHIBAN KK) 24 August 1999 (1999-08-24)

## Description

### FIELD OF THE INVENTION

The invention relates to a spray applicator, the use of such spray applicator and the use of the treatment liquid (that is contained in the spray applicator). The invention further relates to a composition comprising said treatment liquid (that is contained in the spray applicator) for cosmetical and/or medical use. Further the invention relates to a method for providing said spray applicator.

### BACKGROUND OF THE INVENTION

Various compositions are known for the treatment of skin lesions, such as warts, corn and calluses, actinic keratosis, keratosis pilaris, acne, skin hyperpigmentation and/or nail lesions, such as ingrown toenails and/or lesions in mucous membranes, such as cold sores and mouth ulcers. WO2012007584, for instance, describes a composition for the treatment of superficial lesions, in particular skin lesions, mucous membrane lesions and/or nail lesions, an applicator comprising such a composition and the use of such a composition. The composition comprises an effective amount of trichloroacetic acid, at least one thickener, and a physiologically acceptable solvent. The composition is considered effective against a plethora of superficial lesions selected from the group consisting of viral warts, verrucae, water warts (molluscum contagiosum), corns and calluses, and skin hyperpigmentation: age spots, solar lentigo, senial lentigo, acne, keratosis pilaris, actinic keratosis, mouth ulcers (canker sores), cold sores, ingrown toenails, onychomycosis, and eyelid xanthelasma.

WO2007139378 describes a device for cold treating a tissue. The invention also relates to an assembly of such a device and a container comprising a cryogenic liquid, such as a spray can. The invention further relates to a method for cold treating a tissue.

### SUMMARY OF THE INVENTION

Many prior art methods to treat warts or other skin lesions are not effective or not effective enough. Further, some of the prior art methods need specific care and medical supervision, such as the treatment of warts with liquid nitrogen.

Hence, it is an aspect of the invention to provide an alternative method and/or applicator, which preferably further at least partly obviate one or more of the above-described drawbacks, and which is especially safe.

Therefore, the invention provides a spray applicator for topical treatment, the spray applicator comprising a container comprising a treatment liquid under pressure and a propellant, wherein the container is in fluid connection with a spray valve system for application of at least part of the treatment liquid on a topical area, wherein the treatment liquid comprises an acid, wherein the spray applicator further comprises a distance holder configured to arrange on a topical area and comprising an opening configured to enclose a topical lesion, wherein the spray applicator is configured to spray the treatment liquid through the opening in the distance holder, wherein the spray applicator is configured to provide a spray having a spray angle wherein 90% of a spray dose escapes from an outlet of the spray valve system, wherein the spray angle is in the range of 15-45°, as defined in claim 1.

In an aspect, the invention provides a spray applicator for topical treatment, the spray applicator comprising a container comprising a treatment liquid under pressure and a propellant, wherein the container is in fluid connection with a spray valve system, especially a dosage controlled spray valve system, for application of at least part of the treatment liquid on a body surface (herein also indicated as topical area), wherein the treatment liquid comprises an acid, wherein the container comprises a container wall having a top layer and optionally a top layer coating on said top layer. In a specific embodiment, the top layer comprises a metal and the top layer coating comprises in an embodiment a polymer, such as polypropylene or polyethylene, and/or a resin. In yet a further aspect, the invention provides a spray applicator, especially for topical treatment, the spray applicator comprising a container comprising a treatment liquid under pressure and a propellant, wherein the container is in fluid connection with a spray valve system (also comprised by the spray applicator) for application of at least part of the treatment liquid on a topical area, wherein the treatment liquid comprises an acid, wherein the container comprises a container wall which may comprise aluminum or (stainless) steel and a coating comprising a polymer and/or a resin, wherein the spray applicator further especially comprises a distance holder configured to arrange on a topical area and comprising an opening configured to enclose a topical lesion, wherein the spray applicator is configured to spray the treatment liquid through the opening (herein also indicated as "first opening") in the distance holder, especially thereby reducing exposure of topical areas that need not be exposed to the treatment liquid. At least part of such (healthy) topical area is covered by part of the distance holder (the part that surrounds the opening in the distance holder).

Hence, in an embodiment the container comprises a container wall comprising aluminum or (stainless) steel and a coating comprising one or more of a polymer and a resin, wherein the top layer coating comprises one or more of polyethylene and polypropylene, and wherein the metal of the top layer of the wall (thus) comprises aluminum or (stainless) steel.

The invention also provides the above applicator comprising a composition comprising such treatment liquid and the propellant as defined herein, wherein the composition is for use in freezing and acid treatment of a topical lesion, such as in a cosmetic treatment. The invention also provides the above applicator comprising a composition comprising such treatment liquid, wherein the composition is for use in (acid) treatment of a topical lesion, such as in a cosmetic treatment. The invention also provides the above applicator comprising such composition comprising such treatment liquid and the propellant as defined herein, wherein the composition is for use in freezing and acid treatment of a topical lesion, such as in a medical treatment. The invention further provides the above applicator comprising such composition wherein the composition is for use in the treatment of a wart (or corn). Further, the invention also provides the above applicator comprising such treatment liquid per se, wherein the treatment liquid is for use in freezing and acid treatment of a topical lesion, such as in a cosmetic treatment. The invention also provides the above applicator comprising such treatment liquid, wherein the treatment liquid is for use in freezing and acid treatment of a topical lesion, such as in a medical treatment. The invention further provides the above applicator comprising such treatment liquid wherein the treatment liquid is for use in the treatment of a wart (or corn). In a specific embodiment, the composition may further comprise vitamin A. In yet a further embodiment, the treatment liquid further comprises vitamin A.

The acid in the treatment liquid is especially applied to peel the skin or mucous membrane, especially the skin, by which the skin or mucous membrane may renew itself. The propellant is not only used to provide the spray, but may especially advantageously also cool the treatment liquid and/or the topical lesion. Furthermore the propellant is also supporting a penetrating effect into the epidermis of the skin to provide a more in-depth penetration of the treatment liquid. The treatment liquid and the propellant may both have an advantageous effect on the lesion to be treated, such as a wart (or corn): freezing, due to the propellant, may induce blister formation and the acid may have a peeling effect. This may thus lead to a freezing effect, in addition to the acid treatment or acid effect. It appears that this combined treatment is more efficient than the separate treatments alone. Further, the invention may allow a more effective treatment than conventional cryotherapy but may be more efficient than traditional wart acid treatments. The treatment liquid, especially the acid, may provide an oxidation effect to the lesion.

The spray applicator or the treatment liquid, or the treatment liquid in combination with the propellant, may be used for several types of lesions. The spray applicator, or the treatment liquid, or the treatment liquid in combination with the propellant, may be applied for medical use and/or non-medical use, such as cosmetical use. Herein, the term "treatment liquid" refers to the liquid comprising the acid and optionally one or more other components, and the term "treatment composition" refers to the composition or mixture comprising the treatment liquid and the propellant, which is applied on the topical area (and which escapes as spray jet from the spray valve system).

In a further aspect, the invention especially provides the use of the spray applicator, as defined herein, for a cosmetical treatment of a topical lesion as further defined in the accompanying claims. The spray applicator may be used for treatment of a topical lesion selected from the group consisting of epidermodysplasia veruciformis, a HPV (human papillomavirus) caused skin disorder, acne, a scar, a wrinkle, a tattoo, eyelid xanthelasma, an age spot, a senial lentigo, a solar lentigo, a melasma, a hand wart, a plantar wart, a corn, an ingrown toe nail, and a mucous membrane lesion. The term "wart" may e.g. relate to one or more of verruca vulgaris and verruca plantaris. The treatment liquid is especially suitable for the treatment of one or more of a wart and a corn. The invention provides in yet a further aspect also the above applicator comprising the treatment liquid as defined herein, wherein the treatment liquid is for a therapeutic treatment of a topical lesion as further defined in the accompanying claims.

The term topical treatment especially relates to the treatment with the treatment liquid (and propellant) to a body surface, such as the skin or a mucous membrane, especially the (human) skin.

In a specific embodiment, for the spray applicator one or more of the following conditions, especially all, may apply: (i) the acid comprises one or more acids selected from the group consisting of trichloro acetic acid, salicylic acid, formic acid, glycolic acid, dichloro acetic acid, monochloro acetic acid, boric acid, nitric acid, sulfuric acid, phosphoric acid, oxalic acid, lactic acid, and hydrochloric acid; (ii) the propellant comprises one or more propellants selected from the group consisting of dimethyl ether (DME), diethyl ether (DEE), methyl ethyl ether (methoxy ethane), carbon dioxide (CO₂), nitrogen (N₂), nitrous oxide, propane, butane, liquefied petroleum gases (LPG), and a chlorofluorcarbon (CFC); (iii) the top layer coating comprises a polyolefin, especially one or more of polyethylene and polypropylene and/or a resin; and (iv) wherein the metal of the top layer comprises aluminum or (stainless) steel or (stainless) steel. Even more especially, the treatment liquid comprises at least trichloroacetic acid (TCA) and wherein the propellant comprises at least one or more of dimethyl ether (DME), diethyl ether (DEE), and methyl ethyl ether (methoxy ethane). Hence, the acid may also comprise a combination of two or more different acids. A chlorofluorcarbon (CFC) may e.g. be selected from the group consisting of dichlorodifluoromethane (CCl₂F₂) and chlorodifluoromethane (CHClF₂). Further, also the propellant may comprise a combination of two or more propellants.

The top layer coating, when available, or the top layer, in case no coating is available, especially comprises an acid resistant material. Especially good materials for the top layer coating may be selected from the group consisting of chlorinated polyvinyl chloride (CPVC), ethylene chlorotrifluoroethylene (ECTFE), (enhanced) polytetrafluoroethylene, (enhanced PTFE), high-density poly ethylene (HDPE), polyether ether ketone (PEEK), polypropylene (PP), polysulfone (PSU), polyphenylene sulfide (PPS), polyvinyl chloride (especially type 1 (PVC, Type 1) or type 2 (PVC, Type 2)), polyvinylidene fluoride (PVDF), polytetrafluoroethylene (PTFE), Polyamide-imide (PAI), and ultra high molecular weight polyethylene (UHMW). These materials are especially resistant against the acid content of the container. These materials may however also be used as wall of the container (and thus the top layer comprising such material).

The coating(s) (or internal lining(s)) which can be used in combination with or stand alone with the coating may be selected from the group of an epoxy phenolic, a polyamidimid, a polyester and a vinyl organosol.

Especially, the propellant has a boiling point of less than 10 °C, or even less than -20 °C. Further, in a specific embodiment the pressure in the container is in the range of 1.5-80 bar, like 1.5-60 bar, such as especially 1.5-10 bar, like 4-10 bar, such as especially at least 5 bar.

Alternatively, the propellant, or optionally a further component of the treatment liquid, may have a heating effect (on the topical area (such as the skin) to which the treatment composition is applied. Both a propellant having a cooling effect and a propellant having a heating effect (or a further component having such effect), may lead to blister formation. Hence, optionally an exothermic effect on the topical area may be achieved. This (also) facilitates e.g. the removal process of the wart (or corn). For instance, after the application of the treatment liquid, after some time the wart may fall off.

Of course, the treatment liquid may optionally comprise one or more other ingredients, including one or more other active ingredients. The composition as described herein may comprise other ingredients commonly used in cosmetics and pharmaceutical products, such as one or more of a surfactant, a colorant and a perfume.

Trichloroacetic acid (TCA) in the treatment liquid as described herein, proved to be effective against a plethora of skin lesions, in particular warts, corn and calluses, molluscum contagiosum, acne, skin hyperpigmentation, actinic keratosis as well as nail lesions including ingrown toenails and onchyomycosis, and lesions in mucous membranes such as mouth ulcers and cold sores. Application of trichloroacetic acid is particularly effective against genital warts, and especially against ano-genital warts where it has been studied in comparative trials where its effectiveness was compared with other anti-wart therapies. In addition, TCA application was effective in various other skin disorders, in particular: epidermodysplasia veruciformis- skin disorder also caused by HPV virus, acne, to remove acne scars and wrinkles, tattoo removal, eyelid xanthelasma, age spots, senial lentigo and solar lentigo, and melasma.

In a specific embodiment, the acid comprises one or more acids selected from the group consisting of trichloro acetic acid, salicylic acid, glycolic acid, dichloro acetic acid, monochloro acetic acid, boric acid, nitric acid, sulfuric acid, phosphoric acid, oxalic acid, and lactic acid.

Additionally or alternatively, the treatment liquid may comprise one or more of formic acid and salicylic acid, which alone or in combination may also provide a good result. Hence, in an embodiment the treatment liquid comprises TCA, and optionally formic acid, and in another embodiment the treatment liquid comprises formic acid, and optionally TCA.

Salicylic acid has a positive effect in treating skin and nails. In addition to that, salicylic acid gives a mild anaesthetic effect. Salicylic acid in combination with TCA showed a synergistic effect against skin lesions and nail lesions. Another advantage is that using a combination of salicylic acid and TCA allows for a composition with a relatively low concentration of each of salicylic acid and TCA with a similar effect to compositions using only TCA or only salicylic acid. Using the relatively low concentrations of TCA and salicylic acid decreases the chance of skin irritation due to either of these compounds. Moreover, salicylic acid diminished the discomforting burning feeling on skin and nails sometimes experienced by persons treated with products containing substantial amounts of TCA.

The treatment liquid may thus amongst others be used in for instance a skin peeling treatment, for the medical or cosmetic treatment of skin lesion selected from the group consisting of warts, corn and calluses, and for nail treatments including ingrown toenails. A postulated mechanism of action is that the composition comprising TCA, or another acid, softens the skin or nail, and enables to peel the skin or nail lesion away. For severe lesions, multiple treatments may be needed. Both cosmetic and medical treatments may be performed with the compositions comprised in the spray applicator according to the invention. Other active ingredients contributing to the treatment of the lesion may be added. For instance salicylic acid (see also above) is another component effective in corroding the skin or nails, and may be used as an additional active ingredient in combination with trichloroacetic acid. The freezing effect of the propellant seems to enhance these mechanisms, or at least the peeling mechanism, as it facilitates blister formation. Hence, the invention further provides the use of the above applicator for the cosmetic treatment of the human skin, in particular for a cosmetic treatment of a topical lesion selected from the group consisting of a scar, a wrinkle, a tattoo, an age spot, a senial lentigo, a solar lentigo, and a melasma. The invention further provides the above applicator comprising a composition comprising the treatment liquid and the propellant as defined herein wherein the composition is for use in freezing and acid treatment of a topological lesion. Especially, the composition is used for a skin peeling treatment or the treatment of a skin lesion selected from the group consisting of warts, corn and calluses. The compositions are particularly effective against warts and related lesions, including viral warts, verrucae, and water warts (molluscum contagiosum). These treatments are typically considered medical treatments rather than cosmetic treatments. The invention also provides the above applicator comprising a composition as described herein wherein the composition is for the medical treatment of nails. In particular nail deformations (for instance as a result from onchyomycosis) and ingrown toenails may be treated effectively. The composition may also be used for the treatment of lesions in mucous membranes, in particular mouth ulcers and cold sores, or for the treatment of age spots, solar lentigo, senial lentigo, and acne.

The invention further provides the above applicator comprising a treatment liquid wherein the treatment liquid is for the therapeutic treatment of a topical lesion selected from the group consisting of epidermodysplasia veruciformis, a HPV caused skin disorder, acne, eyelid xanthelasma, a hand wart, a plantar wart, a corn, an ingrown toe nail, and a mucose membrane lesion (in particular, mouth ulcers or cold sores).

The treatment liquid especially comprises an aqueous liquid, such as especially water, and the acid. Especially, the total amount of other components in the treatment liquid, other than the acid and the liquid, such as the aqueous liquid, is less than 10 wt.%, especially less than 5 wt.%, examples of other compounds, such as a colorant or a perfume, are mentioned above. Especially, the treatment liquid comprises the acid in a concentration up to 40 wt.%, such as at maximum 35 wt.%, even more especially at maximum 20 wt.%. Higher acid concentrations may lead to undesired topical effects and/or may be unsafe, such as in the kind of application with children or specific topical parts, like mucous membrane. In a specific embodiment, the treatment liquid comprises the acid in a concentration in the range of 1-10 wt.%. Lower concentrations than 1 wt.% may not be effective enough. Especially, the concentration of the acid in the treatment liquid is at least 2 wt.%, even more especially at least 3 wt.%.

The spray applicator allows without contact of the spray valve system of the applicator with the skin, etc., the treatment of the topical lesion. This is safer and also more hygienic than applicators that need contact with the lesion. The spray applicator may especially be configured to provide a single spray dose of in embodiments not more than 1 ml, such as especially not more than 0.75 ml, such as 0.1-0.5 ml, like 0.1-0.2 ml, or less than 0.2 ml. As will be known by the person skilled in the art, this can be tuned by amongst others the nozzle (outlet) dimensions and the pressure of the propellant in the container. A dosage valve or dosage application may be used to control that the desired spray dose is substantially always met. In some instances, especially in view of safety, a narrow spray may be desirable. Hence, the spray applicator is configured to provide a spray having a spray angle (opening angle) (θ) wherein 90% of a spray dose escapes from an outlet of the spray valve system, wherein the spray angle (θ) is in the range of 15-45°. The spray angle may be tuned by the dimensions of the nozzle. For instance, the nozzle (of the spray valve system) may comprise a conical channel.

The applicator is especially configured to provide a pressurized liquid to the skin. The applicator is especially configured to provide a fine stream of pressurized liquid. A high-speed stream of fluid may impact the skin lesion and delivers cold and acid. This pressurized liquid penetrates better into the skin than a non-pressurized. Hence, with the present invention a combination of a cryogenic effect, a peeling effect, and a penetration effect may be obtained. This appears to provide better results than a single effect or a subsequent application of the effects. Temporarily, the skin may cool down to a temperature below 0 °C, even below -5 °C, such as even down to about - 90 °C. Hence, in an embodiment the applicator is configured to provide the treatment liquid to the skin and induce a temporarily cooling of the skin at the topical area where the liquid is applied to a temperature below -5 °C, such as below -20 °C, like in the range of -5 - -50 °C. Furthermore the pressure that is provided by the container can also have beneficial effects due to the fact that the treatment solution is provided deeper in the epidermis. Hence a pressured container is provided, that allows a deeper skin penetration of the treatment liquid than conventional applications and applicators.

In view of safety, especially child safety, the spray valve system may in an embodiment include an infant opening protection. For instance, infant opening protection system may include a turning system that has a state wherein the spray valve system is blocked and a state wherein the spray valve system can be applied.

The container comprises a container wall. The container comprises a top layer or top surface, i.e. the outer part of the wall, which is in physical contact with the treatment liquid and/or propellant when the container is filled. This wall may be entirely of a metal such as aluminum or (stainless) steel. In such instance, the top layer is by definition aluminum or (stainless) steel. Hence, the container wall may be made of aluminum or (stainless) steel. On top of this top layer, especially in view of life time of the spray applicator, the container wall comprises a coating. It appears that a polymer coating, especially selected from the group consisting of polypropylene and polyethylene, provides good properties. The coating may include a single-layer coating or a multi-layer coating. In case of the latter coating, adjacent layers of the multi-layer coating may differ in composition and/or thickness. The polypropylene polymer may e.g. be selected from the type of homopolymer, random copolymer, and block copolymer. The comonomer is typically used with ethylene. Likewise, the polyethylene polymer may e.g. be selected from the type of homopolymer, random copolymer, and block copolymer. Examples of PE may e.g. be selected from the group consisting of ultra-high-molecular-weight polyethylene (UHMWPE), high-density polyethylene (HDPE), cross-linked polyethylene (PEX or XLPE), medium-density polyethylene (MDPE), linear low-density polyethylene (LLDPE), low-density polyethylene (LDPE), and very-low-density polyethylene (VLDPE). Hence, the container wall may comprise a metal (layer), especially aluminum or (stainless) steel, and an inner surface coating comprising a polymer coating. However, alternatively, the container wall may comprise a polymer and/or resin, such as one of the above indicated polymers, or one or more of the above indicated acid resistant polymers. In a further embodiment, the container wall comprises a polymer and a polymer and/or resin top coating, such as one or more of the above indicated acid resistant polymers.

In a further embodiment, the spray applicator comprises a distance holder configured to arrange on a topical area, such as the skin, wherein the distance holder comprises an opening configured to enclose a topical lesion. The user may thus arrange the spray applicator on the skin with the opening surrounding the skin lesion. In this way, exposure of healthy skin to the treatment liquid may be minimized, but hereby it may also be prevented that the spray valve system comes into contact with the topical lesion (such contact is not desirable). The distance holder is an element that can be used to arrange the spray applicator to a topical area with a (more) controlled distance from the spray valve system to the topical lesion to be sprayed with the treatment liquid. In this way, the dosage the lesion receives can be better controlled than with a manual arrangement of the spray applicator over the lesion, in addition the distance will also contribute to the effectiveness since the mixture needs to vaporize in order to have a cooling effect. Hence, especially the spray applicator is configured to spray the treatment liquid through the opening in the distance holder. Further, the distance holder is configured to prevent physical contact between the spray valve system and the topical area. Optionally, the opening of the distance holder is adjustable in size. For instance, the distance holder may include an adjustable diaphragm (as opening). In this way, even more the healthy topical area may be protected and efficient and targeted application of treatment liquid to the desired skin lesion is ensured. The distance holder may be integrated in the spray applicator, or may be an attachable unit, e.g. with Snap-On snap-off elements. The distance holder may include a transparent part, that facilitates visual inspection by a user of the area that is sprayed. Hence, the applicator may be designed to ensure a visual control during the use of the applicator to ensure a safe and effective treatment. Alternatively or additionally, the distance holder may be configured in such a way that a user may visual inspect the area that is sprayed when used by the user himself or herself. The opening in the distance holder may be round or square or oval, or have another shape. Especially, part of the distance holder is configured to fit on a human body. Optionally, a plurality of distance holders may be provided, which may fit to different parts of the body.

The transparent part may include one or more of a transparent material and an opening. In an embodiment, the distance holder has a conical shape. In yet another embodiment, the distance holder has a cylindrical shape. In an embodiment, the distance holder comprises a hollow body, with a wall of the body configured for instance to provide the conical or cylindrical shape, with the wall comprising the transparent part. The wall may be entirely of a transparent material. However, the wall may also include one or more openings. Such openings may be used for one or more of visual inspection and influx of air, entrained with the spray. This influx of air also appears to have a beneficial effect on especially the peeling effect of the acid. Further, the distance holder is especially configured to protect the healthy skin surrounding the topical lesion. Hence, the distance holder may especially be configured to provide a channel with a wall configured to provide that channel and configured to bridge a distance between the spray applicator and a topical area of a user, wherein the wall is further especially configured to circumferentially surround the spray during spraying of the treatment liquid. Hence, the wall may especially be configured to substantially allow the treatment liquid escaping from the distance holder only via the opening in the distance holder. Therefore, the distance holder may in embodiments essentially comprise a first opening configured to enclose a topical lesion and a second opening for entrance of the treatment liquid or at least part of the spray valve system, wherein the wall is configured to provide a channel with the first opening at one side and a second opening at an opposite side, wherein the wall may optionally include further openings, such as for influx of air. Especially the ratio of the cumulative (smallest) cross-sectional areas of these openings to the area of the wall is less than 3:1, such as less than 2:1, like less than 1:1, or even less than 0.1:1. Here the term "smallest cross-sectional area" may be applied, as the openings in the wall may optionally have tapered shapes. The distance holder including said wall may especially have a tapered shape or a cylindrical shape, and may optionally consist of a transparent material, such as PMMA, siloxanes, etc.. Hence, in an embodiment the distance holder (120) includes a transparent part. Further, especially the distance holder may comprise the (first) opening and a second opening for entrance of the treatment liquid or at least part of the spray valve system, and a wall configured to provide a channel with the opening at one side and the second opening at an opposite side. Further, the wall may optionally include one or more openings.

In an embodiment, the treatment liquid may further comprise vitamin A. Vitamin A is a retinoid, which may act to affect HPV induced lesions to prevent recurrence of the virus. Vitamin A may evoke or potentiate immune response to warts and thus in combination with the described composition, treatment may be more effective when vitamin A is included. Hence, vitamin A may promote healing and/or prevent reoccurrence of the skin lesion, especially warts.

In yet a further aspect, the invention also provides a method of providing the spray applicator, especially as defined in the accompanying claims, the method comprising:
- premixing acid and an aqueous liquid and providing the mixture to the container;
- providing the propellant in a liquid phase to the container; and
- closing the container with the spray valve system (and arrange a manual applicator to the spray valve system).

Further, the invention provides a kit of parts, as further defined in the accompanying claims, comprising a container with a spray valve system as defined herein, and a distance holder, especially a plurality of distance holders having differently sized openings. Each of the distance holders is especially configured to be functionally coupled to the container with spray valve system to provide said spray applicator. The kit of parts may further comprise a patch comprising a porous part with pores. The treatment liquid, especially under pressure, may penetrate the porous part and come into contact with the skin lesion. The patch may be a further protection means and/or dosage means.

The term "substantially" herein, such as in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of'. The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of' but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices herein are amongst others described during operation. As will be clear to the person skilled in the art, the invention is not limited to methods of operation or devices in operation.

Use of the verb "to comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

The various aspects discussed in this patent can be combined in order to provide additional advantages.

### BRIEF DESCRIPTION OF THE DRAWINGS

Embodiments of the invention will now be described, by way of example only, with reference to the accompanying schematic drawings in which corresponding reference symbols indicate corresponding parts, and in which:

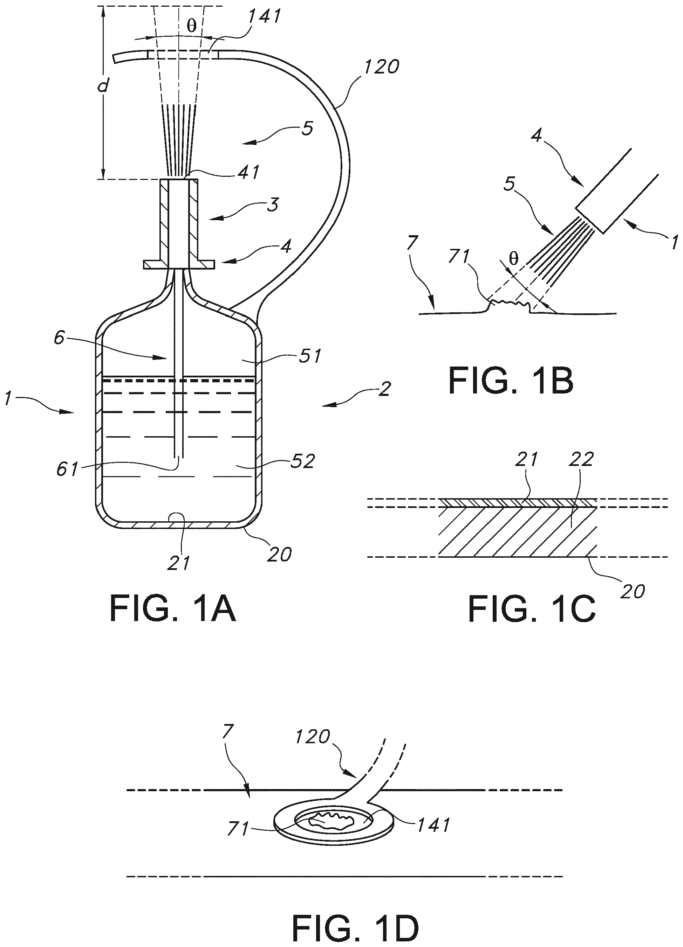

Figs.1a-1d schematically depict embodiments and aspects of the spray applicator.

The drawings are not necessarily on scale.

### DETAILED DESCRIPTION OF THE EMBODIMENTS

Fig. 1a schematically depicts an embodiment of the spray applicator 1. The spray applicator is especially an aerosol spray applicator. The spray applicator 1 comprises a can or container 2 as well as a spray valve system 4 with a nozzle or outlet 41. The container 2 comprises a container wall 20, which may optionally include a container wall coating 21. Further, optionally integrated with the valve system 4, the spray system 4 comprises a manual applicator. For instance by pressing this manual applicator, the spray valve systems frees treatment liquid 52 from the container 2 which is under elevated pressure due to the presence of a propellant under pressure. The spray valve system 4 has an opening or nozzle 41. The propellant is indicated with reference 51. Part of the propellant may also be in the liquid phase. The spray applicator may further comprise a dip tube 6, in fluid connection with the spray valve system 4 and with a dip tube opening 61, which during most of the lifetime of the spray applicator 1 will be emerged in the treatment liquid.

In an embodiment, the spray applicator 1 may especially be configured to provide a single spray dose of not more than 0.75 ml. Further, the spray applicator 1 is configured to provide a spray 5 having a spray angle θ wherein 90% of a spray dose escapes from an outlet 41 of the spray valve system 4, wherein the spray angle θ is in the range of 15-45°. These spray characteristics may especially be obtained within a distance d of the nozzle 41, such as within a distance of 0-5 cm, like 0.5-5 cm, such as 0.5-2 cm. The spray valve system may include a metering valve. Especially, the spray valve system 4 is configured to administer the treatment liquid in a metered manner.

The spray applicator 1, according to the invention, includes a distance holder 120, which includes an opening 141 (herein also indicated as "first opening"). With such applicator, the opening may be arranged around the wart, and the jet may only be provided to the topical area of the opening (see also Fig. 1d). Optionally, the distance holder includes transparent regions, such as openings or transparent material, thereby facilitating the user to easier perceive the topical area that is (to be) treated.

Fig. 1a is a very schematic embodiment. Of course, the spray applicator may include a container in a holder. Such holder may for instance host the distance holder 120. The distance holder 120 may be integrated in the spray applicator 1 or may be temporarily connected to the spray applicator. Optionally the distance holder 120 is bendable and can be arranged in a position for use and a position for rest. The former position may e.g. provide a smaller spray applicator (for transport purposes).

In the schematic drawing of 1a, the distance holder 120 does not entirely circumferentially surround the spray 5 during application.

Fig. 1b schematically depicts how the spray applicator 1 can be used. For the sake of simplicity, only the valve system 4 with nozzle 41 is shown. The spray or spray jet 5 may be applied to a lesion 71, such as a wart, on a body surface or topical area 7, such as the skin.

Fig. 1c schematically depicts part of the container wall of the container. The container wall 20 may optionally comprise a container wall coating 21. This coating will thus be in contact with the treatment liquid. A top layer of the container wall 20 is indicated with reference 22. The container wall may e.g. be of aluminum or (stainless) steel and thus the top layer 22 is of aluminum or (stainless) steel. On the top layer 22, optionally the coating 21 may be available, for e.g. a PP coating.

Fig. 1d schematically depicts how the distance holder 120 can be arranged to a topical region or body surface 7, such as the skin, with the skin lesion 71, such as a wart surrounded by the opening 141. This allows dosing the treatment liquid to the opening only, thus the topical region in the opening only, and substantially not to healthy topical regions. Optionally, the opening is scalable, i.e. the opening area may be adapted by the user. For instance a diaphragm type of opening may be used.

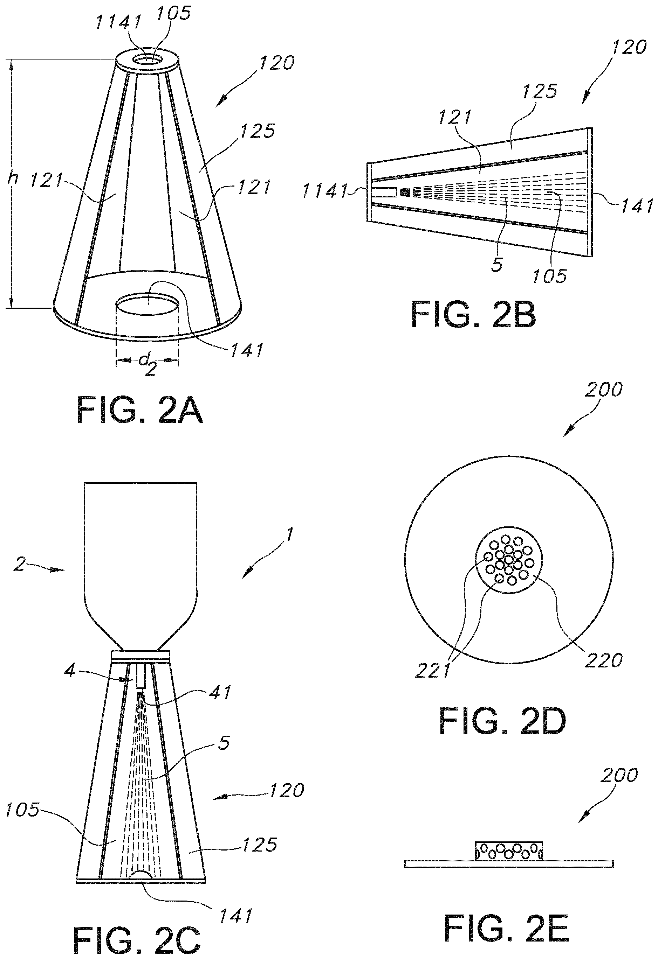

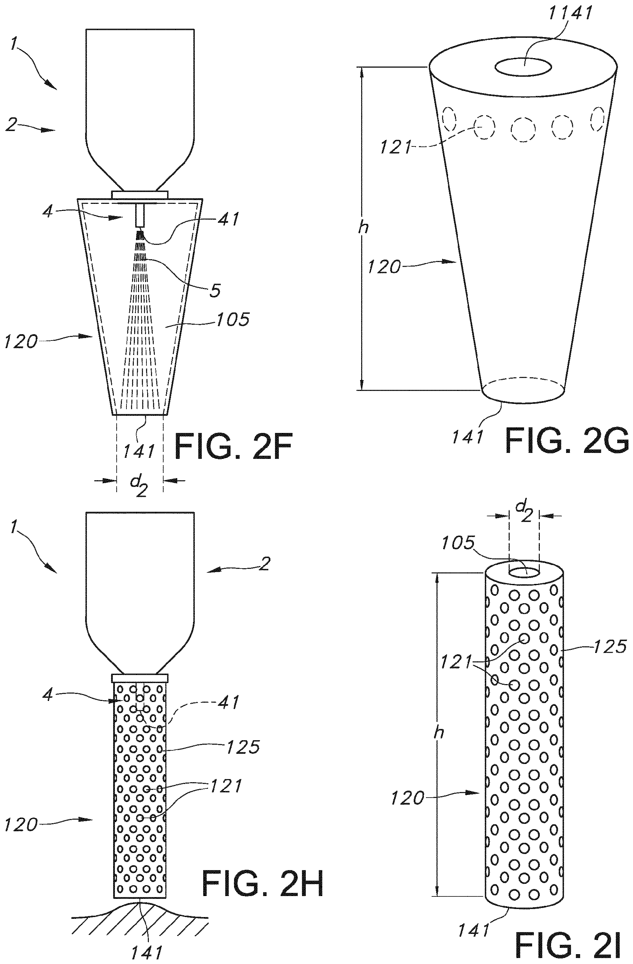

Figs. 2a-2c and 2f-2i schematically depict embodiments of distance holders 120 that substantially surround a spray during use. Referring to Figs. 2a-2c the distance holder has a conical shape with a first opening 141 configured to enclose a topical lesion. Further, the distance holder comprises a second opening 1141 configured to receive at least part of the spray valve system, especially at least to circumferentially surround the opening or nozzle 41 of the spray valve system 4. Reference 125 indicates the wall of the distance holder 125 and reference 121 indicates the opening(s) in the wall of the distance holder 120. Especially the ratio of the cumulative (smallest) cross-sectional areas of these openings to the area of the wall is less than 3:1, such as less than 2:1, like less than 1:1, or even less than 0.1:1. For instance, referring to the cone of Fig. 2a, the (external) area of the openings 121 relative to the wall (parts) 125 may be in the range of about 2:1. Reference 5 indicates the channel that is provided by wall 125 and through which the spray may be provided. The distance holder may have a height h in the range of 0.5-10 cm, such as especially 0.5-5 cm, such as 1-5 cm or for instance 0.5-2 cm. The first opening 141 is schematically depicted as circularly shaped. However, the shape may also be different. The diameter of the opening is indicated with reference d2. The equivalent circular diameter, i.e. the diameter as if the first opening is circular, may be in the range of 0.2-5 cm, such as in the range of 0.5-4 cm.

Figs. 2d-2e schematically depict a patch 200 that may be configured over the topical area, with a porous part 220 with pores 221 over the topical lesion. The pores 221 are configured to allow penetration of the treatment liquid. Fig. 2e is a side view of an embodiment. Hence, in an embodiment, plasters or other patches, with e.g. a sponge membrane, may be applied to apply the liquid to the topical lesion. The treatment liquid is provided through the porous part, such as a membrane, like a sponge membrane. When using the distance holder, the opening 141 of the distance holder can be placed on the patch 200 over at least part of the porous part 220.

Figs. 2f-2g schematically depicts a distance holder 120 similar to the distance holder schematically depicted in Figs. 2a-2c. However, now the distance holder has conical shape which is opposite. Further, here the distance holder 120 has a wall 125 that is closed, though in Fig. 2g optional openings 121 are depicted. Such openings 121 may be configured closer to the second inlet 1141 than to the first inlet 141.

Figs. 2h-2i schematically depict an embodiment of the distance holder having substantially cylindrical shaped wall 125, with a plurality of relative small openings 125.

In a further embodiment, the invention also provides a kit of parts comprising a container 2 with a spray valve system 4 and a plurality of distance holders 120 having differently sized (first) openings 141. Each distance holders 120 can functionally be coupled to the container with spray valve system to provide the above described spray applicator 1. With different sized openings, for instance to be used depending on wart size, dosage may also be controlled. The size of the opening 141 may also determine how many sprays of a treatment liquid should be administered. Referring to Figs. 2a-2c and 2f-2i, optionally the first opening 141 is adjustable in size.

Hence, the invention provides amongst others a composition for treatment of skin lesions by using a special designed (1) coated aluminum or (stainless) steel container, (2) a dosage delivery valve and nozzle, (3) a targeted applicator, (4) an active medical serum (acid + freezing agent), and optionally (5) a child safety lock. For instance, a composition for treatment of superficial lesions may be provided, wherein a freezing agent is applied having a low boiling point and an (extreme) skin exfoliating acid, such as acetic acid. Further, the invention provides a device for the application of the active serum on the skin lesion, wherein the device comprises an applicator to include a dosage delivery nozzle to apply, in an acid compliant coated aluminum or (stainless) steel container consisting of a (tubular) container and (cylindrical) body with a targeted open applicator which ensures that the dosage valve is released at a certain distance from the skin to allow oxidization for controlled temperature of the freezing agent, along with the active serum to treat the skin lesion. TCA may advantageously be provided as powder, which can be combined with a liquid to provide the treatment liquid.

Experimental work was done wherein the effectivity of acids on warts, the effectivity of cryogenic treatment of warts, and the effectivity of the combination of acids and cryogenic treatment of warts in one dosage, as described above, was evaluated.

**Table 1. Analysis of combination therapy of 30% TCA with cryogen**

| Sex | Age | Weeks to Resolution | Applications of 30% TCA with cryogen (-57°C) |
|---|---|---|---|
| F | 10 | 4 | 4 |
| M | 11 | Unresolved | 17 |
| M | 7 | 3 | 3 |
| M | 7 | 3 | 3 |
| M | 7 | 3 | 3 |
| M | 7 | 3 | 3 |
| M | 7 | 3 | 3 |
| F | 8 | 9 | 9 |
| M | 15 | 8 | 7 |
| M | 15 | 8 | 7 |
| M | 15 | 8 | 7 |
| M | 15 | 16 | 15 |
| M | 15 | Unresolved | 20 |
| F | 12 | 3 | 3 |
| F | 12 | 2 | 3 |
| F | 12 | 2 | 3 |
| F | 12 | 7 | 3 |
| F | 12 | 7 | 3 |
| M | 8 | 16 | 11 |
| F | 8 | 8 | 7 |
| F | 8 | 8 | 7 |
| F | 6 | 6 | 5 |
| F | 6 | 6 | 5 |
| F | 6 | 6 | 5 |
| M | 51 | 3 | 3 |
| F | 15 | 9 | 8 |

Of the 28 treated warts, 93% resolved successfully via this combination method over an average of 5.4 weeks and 5.96 acid-cryogen applications. The combined cryogen/acid treatment method can be seen as a low-risk, efficient, gentle and highly successful method to treat warts (and other topical lesions). The results are in line with the assumptions of combining the two modes of action of an acid (peeling of the skin) and a cryogen (destruction of HPV infected cells).

## Claims

1. A spray applicator (1) for topical treatment, the spray applicator (1) comprising a container (2) comprising a treatment liquid (52) under pressure and a propellant (51), wherein the container (2) is in fluid connection with a spray valve system (4) for application of at least part of the treatment liquid (52) on a topical area, wherein the treatment liquid (52) comprises an acid, wherein the spray applicator (1) further comprises a distance holder (120) configured to arrange on a topical area and comprising an opening (141) configured to enclose a topical lesion, wherein the spray applicator is configured to spray the treatment liquid (52) through the opening (141) in the distance holder (120), wherein the spray applicator (1) is configured to provide a spray (5) having a spray angle (θ) wherein 90% of a spray dose escapes from an outlet (41) of the spray valve system (4), wherein the spray angle (θ) is in the range of 15-45°.

2. The spray applicator (1) according to claim 1, wherein the treatment liquid (52) at least comprises trichloro acetic acid.

3. The spray applicator (1) according to claim 1, wherein
- the acid comprises one or more acids selected from the group consisting of trichloro acetic acid, salicylic acid, formic acid, glycolic acid, dichloro acetic acid, monochloro acetic acid, boric acid, nitric acid, sulfuric acid, phosphoric acid, oxalic acid, lactic acid, and hydrochloric acid,
- the propellant comprises one or more propellants selected from the group consisting of dimethyl ether, diethyl ether, methyl ethyl ether, carbon dioxide, nitrogen, nitrous oxide, propane, butane, liquefied petroleum gases, and a chlorofluorcarbon,
- wherein the container (2) comprises a container wall (20) comprising aluminum or steel and a coating (21) comprising one or more of a polymer and a resin, wherein the top layer coating (21) comprises one or more of polyethylene and polypropylene; and
- wherein a metal of a top layer (22) of the wall (20) comprises aluminum or steel.

4. The spray applicator (1) according to any one of the preceding claims, wherein the treatment liquid comprises at least trichloroacetic acid and wherein the propellant comprises at least one or more of dimethyl ether, diethyl ether, and methyl ethyl ether.

5. The spray applicator (1) according to any one of the preceding claims, wherein the spray valve system (4) includes an infant opening protection, wherein the propellant has a boiling point of less than 10 °C, and wherein the pressure in the container (2) is in the range of at least 5 bar.

6. The spray applicator (1) according to any one of the preceding claims, wherein the treatment liquid (52) comprises the acid in a concentration up to 40 wt.%, especially wherein the treatment liquid (52) comprises the acid in a concentration in the range of 1-10 wt.%.

7. The spray applicator (1) according to any one of the preceding claims, wherein the spray applicator (1) is configured to provide a single spray dose of not more than 0.75 ml.

8. The spray applicator (1) according to any one of the preceding claims, wherein the distance holder (120) comprises said opening (141) and a second opening (1141) for entrance of the treatment liquid (52) or at least part of the spray valve system (4), and a wall (125) configured to provide a channel (105) with the opening (141) at one side and the second opening (1141) at an opposite side, wherein (i) the wall (125) includes one or more openings (121) or (ii) the distance holder (120) includes a transparent part.

9. The spray applicator (1) according to any one of the preceding claims, wherein the distance holder is configured to prevent physical contact between the spray valve system (4) and the topical area, wherein the opening (141) is adjustable in size.

10. The spray applicator (1) according to any one of the preceding claims, wherein the treatment liquid further comprises vitamin A.

11. Use of the spray applicator (1) according to any one of the preceding claims for a cosmetical treatment of a topical lesion selected from the group consisting of a scar, a wrinkle, a tattoo, an age spot, a senial lentigo, a solar lentigo, and a melasma.

12. The spray applicator (1) according to any of the claims 1-10, comprising the treatment liquid (52), as defined in any one of claims 1-10, wherein the treatment liquid is for the therapeutic treatment of a topical lesion selected from the group consisting of epidermodysplasia veruciformis, a HPV caused skin disorder, acne, eyelid xanthelasma, a hand wart, a plantar wart, a corn, an ingrown toe nail, and a mucous membrane lesion.

13. The spray applicator (1) according to any of the claims 1-10, comprising the composition comprising the treatment liquid (52) and the propellant, as defined in any one of claims 1-10, wherein the composition is for use in freezing and acid treatment of a topical lesion.

14. The spray applicator (1) according to claim 13, comprising the composition as defined in claim 13, wherein the composition is for use in the treatment of a wart or a corn.

15. A method of providing the spray applicator (1) according to any of the claims 1-10, the method comprising:
- premixing acid and an aqueous liquid and providing the mixture to the container (2);
- providing the propellant in a liquid phase to the container (2); and
- closing the container with the spray valve system (4).

16. A kit of parts comprising a container (2) with a spray valve system (4) as defined in any one of the preceding claims 1-10, and a distance holder (120) or a plurality of distance holders (120) having differently sized openings (141), as defined in any one of the preceding claims 1-10.

17. The kit of parts according to claim 16, further comprising a patch (200) comprising a porous part (220) with pores (221).

## Patentansprüche

1. Sprühapplikator (1) zur topischen Behandlung, wobei der Sprühapplikator (1) einen Behälter (2) umfasst, der eine unter Druck stehende Behandlungsflüssigkeit (52) und ein Treibmittel (51) umfasst, wobei der Behälter (2) mit einem Sprühventilsystem (4) zum Aufbringen mindestens eines Teils der Behandlungsflüssigkeit (52) auf eine topische Fläche in Fluidkommunikation steht, wobei die Behandlungsflüssigkeit (52) eine Säure umfasst, wobei der Sprühapplikator (1) weiter einen Abstandshalter (120) umfasst, der konfiguriert ist, sich auf einer topischen Fläche anzuordnen, und eine Öffnung (141) umfasst, die konfiguriert ist, eine topische Läsion zu umschließen, wobei der Sprühapplikator konfiguriert ist, die Behandlungsflüssigkeit (52) durch die Öffnung (141) in dem Abstandshalter (120) zu sprühen, wobei der Sprühapplikator (1) konfiguriert ist, ein Spray (5) mit einem Sprühwinkel (θ) bereitzustellen, wobei 90 % einer Sprühdosis aus einem Auslass (41) des Sprühventilsystems (4) austritt, wobei der Sprühwinkel (θ) im Bereich von 15-45° liegt.

2. Sprühapplikator (1) nach Anspruch 1, wobei die Behandlungsflüssigkeit (52) mindestens Trichloressigsäure umfasst.

3. Sprühapplikator (1) nach Anspruch 1, wobei
- die Säure eine oder mehrere Säuren umfasst, die ausgewählt sind aus der Gruppe, bestehend aus Trichloressigsäure, Salizylsäure, Ameisensäure, Glykolsäure, Dichloressigsäure, Monochloressigsäure, Borsäure, Salpetersäure, Schwefelsäure, Phosphorsäure, Oxalsäure, Milchsäure und Salzsäure,
- das Treibmittel ein oder mehrere Treibmittel umfasst, die ausgewählt sind aus der Gruppe, bestehend aus Dimethylether, Diethylether, Methylether, Kohlendioxid, Stickstoff, Distickstoffoxid, Propan, Butan, Flüssiggasen und einem Fluorchlorkohlenwasserstoff,
- wobei der Behälter (2) eine Aluminium oder Stahl umfassende Behälterwand (20) und eine ein oder mehrere Polymere und ein Harz umfassende Beschichtung (21) umfasst, wobei die Deckschichtbeschichtung (21) eines oder mehrere von Polyethylen und Polypropylen umfasst; und
- wobei ein Metall einer Deckschicht (22) der Wand (20) Aluminium oder Stahl umfasst.

4. Sprühapplikator (1) nach einem der vorstehenden Ansprüche, wobei die Behandlungsflüssigkeit mindestens Trichloressigsäure umfasst und wobei das Treibmittel mindestens eines oder mehrere von Dimethylether, Diethylether und Methylethylether umfasst.

5. Sprühapplikator (1) nach einem der vorstehenden Ansprüche, wobei das Sprühventilsystem (4) eine Kindersicherung für das Öffnen einschließt, wobei das Treibmittel einen Siedepunkt von weniger als 10 °C aufweist und wobei der Druck in dem Behälter (2) im Bereich von mindestens 5 bar liegt.

6. Sprühapplikator (1) nach einem der vorstehenden Ansprüche, wobei die Behandlungsflüssigkeit (52) die Säure in einer Konzentration von bis zu 40 Gew.-% umfasst, wobei insbesondere die Behandlungsflüssigkeit (52) die Säure in einer Konzentration im Bereich von 1-10 Gew.-% umfasst.

7. Sprühapplikator (1) nach einem der vorstehenden Ansprüche, wobei der Sprühapplikator (1) konfiguriert ist, eine Einzelsprühdosis von nicht mehr als 0,75 ml bereitzustellen.

8. Sprühapplikator (1) nach einem der vorstehenden Ansprüche, wobei der Abstandshalter (120) die Öffnung (141) und eine zweite Öffnung (1141) für den Eintritt der Behandlungsflüssigkeit (52) oder mindestens einen Teil des Sprühventilsystems (4) und eine Wand (125), die konfiguriert ist, einen Kanal (105) mit der Öffnung (141) auf einer Seite und der zweiten Öffnung (1141) auf einer gegenüberliegenden Seite bereitzustellen, umfasst, wobei (i) die Wand (125) eine oder mehrere Öffnungen (121) einschließt oder (ii) der Abstandshalter (120) ein durchsichtiges Bauteil einschließt.

9. Sprühapplikator (1) nach einem der vorstehenden Ansprüche, wobei der Abstandshalter konfiguriert ist, physischen Kontakt zwischen dem Sprühventilsystem (4) und der topischen Fläche zu verhindern, wobei die Öffnung (141) größeneinstellbar ist.

10. Sprühapplikator (1) nach einem der vorstehenden Ansprüche, wobei die Behandlungsflüssigkeit weiter Vitamin A umfasst.

11. Verwendung des Sprühapplikators (1) nach einem der vorstehenden Ansprüche für eine kosmetische Behandlung einer topischen Läsion, die ausgewählt ist aus der Gruppe, bestehend aus einer Narbe, einer Falte, einem Tattoo, einem Altersfleck, einer Lentigo senilis, einer Lentigo solaris und einem Melasma.

12. Sprühapplikator (1) nach einem der Ansprüche 1-10, umfassend die Behandlungsflüssigkeit (52), wie in einem der Ansprüche 1-10 definiert, wobei die Behandlungsflüssigkeit der therapeutischen Behandlung einer topischen Läsion dient, die ausgewählt ist aus der Gruppe, bestehend aus Epidermodysplasia veruciformis, einer durch HPV ausgelösten Hautkrankheit, Akne, Augenlied-Xanthelasma, einer harten Warze, einer Plantarwarze, einem Hühnerauge, einem eingewachsenen Zehennagel und einer Schleimhautläsion.

13. Sprühapplikator (1) nach einem der Ansprüche 1-10, umfassend die Zusammensetzung, die die Behandlungsflüssigkeit (52) und das Treibmittel, wie in einem der Ansprüche 1-10 definiert, umfasst, wobei die Zusammensetzung der Verwendung beim Einfrieren und Säurebehandeln einer topischen Läsion dient.

14. Sprühapplikator (1) nach Anspruch 13, umfassend die Zusammensetzung, wie in Anspruch 13 definiert, wobei die Zusammensetzung der Verwendung bei der Behandlung einer Warze oder eines Hühnerauges dient.

15. Verfahren zur Bereitstellung des Sprühapplikators (1) nach einem der Ansprüche 1-10, wobei das Verfahren Folgendes umfasst:
- Vormischen von Säure und einer wässrigen Flüssigkeit und Bereitstellen der Mischung an den Behälter (2);
- Bereitstellen des Treibmittels in einer flüssigen Phase an den Behälter (2); und
- Schließen des Behälters mit dem Sprühventilsystem (4).

16. Bauteil-Kit, umfassend einen Behälter (2) mit einem Sprühventilsystem (4), wie in einem der vorstehenden Ansprüche 1-10 definiert, und einem Abstandshalter (120) oder einer Vielzahl von Abstandshaltern (120), aufweisend unterschiedlich dimensionierte Öffnungen (141), wie in einem der vorstehenden Ansprüche 1-10 definiert.

17. Bauteil-Kit nach Anspruch 16, weiter umfassend ein Pflaster (200), das ein poröses Bauteil (220) mit Poren (221) umfasst.

## Revendications

1. Applicateur de pulvérisation (1) pour un traitement topique, l'applicateur de pulvérisation (1) comprenant un récipient (2) comprenant un liquide de traitement (52) sous pression et un propulseur (51), dans lequel le récipient (2) est en connexion fluidique avec un système de valve de pulvérisation (4) pour l'application d'au moins une partie du liquide de traitement (52) sur une zone topique, dans lequel le liquide de traitement (52) comprend un acide, dans lequel l'applicateur de pulvérisation (1) comprend en outre un écarteur (120) configuré pour être disposé sur une zone topique et comprenant une ouverture (141) configurée pour enceindre une lésion topique, dans lequel l'applicateur de pulvérisation est configuré pour pulvériser le liquide de traitement (52) à travers l'ouverture (141) dans l'écarteur (120), dans lequel l'applicateur de pulvérisation (1) est configuré pour fournir une pulvérisation (5) ayant un angle de pulvérisation (θ) dans lequel 90% d'une dose de pulvérisation s'échappe d'une sortie (41) du système de valve de pulvérisation (4), dans lequel l'angle de pulvérisation (θ) se trouve dans la plage de 15 à 45°.

2. Applicateur de pulvérisation (1) selon la revendication 1, dans lequel le liquide de traitement (52) comprend au moins de l'acide trichloroacétique.

3. Applicateur de pulvérisation (1) selon la revendication 1, dans lequel
- l'acide comprend un ou plusieurs acides sélectionnés dans le groupe constitué par l'acide tricholoroacétique, l'acide salicylique, l'acide formique, l'acide glycolique, l'acide dichloroacétique, l'acide monochloroacétique, l'acide borique, l'acide nitrique, l'acide sulfurique, l'acide phosphorique, l'acide oxalique, l'acide lactique, et l'acide chlorhydrique,
- le propulseur comprend un ou plusieurs propulseurs sélectionnés dans le groupe constitué par l'éther diméthylique, l'éther diéthylique, le méthyl éthyl éther, le dioxyde de carbone, l'azote, l'oxyde nitreux, le propane, le butane, les gaz de pétrole liquéfiés (GPL), et un chlorofluorocarbure (CFC),
- dans lequel le récipient (2) comprend une paroi de récipient (20) comprenant de l'aluminium ou de l'acier et un revêtement (21) comprenant un ou plusieurs d'un polymère et d'une résine, dans lequel le revêtement de couche supérieure (21) comprend un ou plusieurs du polyéthylène et du polypropylène ; et
- dans lequel un métal d'une couche supérieure (22) de la paroi (20) comprend de l'aluminium ou de l'acier.

4. Applicateur de pulvérisation (1) selon l'une quelconque des revendications précédentes, dans lequel le liquide de traitement comprend au moins de l'acide trichloracétique et dans lequel le propulseur comprend au moins un ou plusieurs de l'éther diméthylique, de l'éther diéthylique, et du méthyl éthyl éther.

5. Applicateur de pulvérisation (1) selon l'une quelconque des revendications précédentes, dans lequel le système de valve de pulvérisation (4) inclut une protection d'ouverture pour nourrissons, dans lequel le propulseur a un point d'ébullition inférieur à 10 °C, et dans lequel la pression du récipient (2) se trouve dans la plage d'au moins 5 bars.

6. Applicateur de pulvérisation (1) selon l'une quelconque des revendications précédentes, dans lequel le liquide de traitement (52) comprend l'acide en une concentration pouvant atteindre 40% en poids, notamment dans lequel le liquide de traitement (52) comprend l'acide en une concentration dans la plage de 1 à 10 % en poids.

7. Applicateur de pulvérisation (1) selon l'une quelconque des revendications précédentes, dans lequel l'applicateur de pulvérisation (1) est configuré pour fournir une dose de pulvérisation unique pas supérieure à 0,75 ml.

8. Applicateur de pulvérisation (1) selon l'une quelconque des revendications précédentes, dans lequel l'écarteur (120) comprend ladite ouverture (141) et une seconde ouverture (1141) pour l'entrée du liquide de traitement (52) ou au moins une partie du système de valve de pulvérisation (4), et une paroi (125) configurée pour fournir un canal (105) avec l'ouverture (141) au niveau d'un côté et la seconde ouverture (1141) au niveau d'un côté opposé, dans lequel (i) la paroi (125) inclut une ou plusieurs ouvertures (121) ou (ii) l'écarteur (120) inclut une partie transparente.

9. Applicateur de pulvérisation (1) selon l'une quelconque des revendications précédentes, dans lequel l'écarteur est configuré pour empêcher le contact physique entre le système de valve de pulvérisation (4) et la zone topique, dans lequel l'ouverture (141) est de taille ajustable.

10. Applicateur de pulvérisation (1) selon l'une quelconque des revendications précédentes, dans lequel le liquide de traitement comprend en outre de la vitamine A.

11. Utilisation de l'applicateur de pulvérisation (1) selon l'une quelconque des revendications précédentes pour un traitement cosmétique d'une lésion topique sélectionnée à partir du groupe constitué par une cicatrice, une ride, un tatouage, une tache de vieillesse, un lentigo sénile, un lentigo solaire, et un mélasme.

12. Applicateur de pulvérisation (1) selon l'une quelconque des revendications 1 à 10, comprenant le liquide de traitement (52), tel que défini dans l'une quelconque des revendication 1 à 10, dans lequel le liquide de traitement est destiné au traitement thérapeutique d'une lésion topique sélectionnée à partir du groupe constitué par l'épidermodysplasie verruciforme, un trouble cutané provoqué par un HPV, l'acné, le xanthélasma des paupières, une verrue palmaire, une verrue plantaire, un cor, un ongle de pied incarné, une lésion d'une membrane muqueuse.

13. Applicateur de pulvérisation (1) selon l'une quelconque des revendications 1 à 10, comprenant la composition comprenant le liquide de traitement (52) et le propulseur, tel que défini dans l'une quelconque des revendications 1 à 10, dans lequel la composition est destinée à une utilisation pour congeler et traiter à l'acide une lésion topique.

14. Applicateur de pulvérisation (1) selon la revendication 13, comprenant la composition telle que définie dans la revendication 13, dans lequel la composition est destinée à être utilisée dans le traitement d'une verrue ou d'un cor.

15. Procédé de fourniture de l'applicateur de pulvérisation (1) selon l'une quelconque des revendications 1 à 10, le procédé comprenant :
- le prémélange d'acide et d'un liquide aqueux et la fourniture du mélange au récipient (2) ;
- la fourniture du propulseur en phase liquide au récipient (2) ; et
- la fermeture du récipient avec le système de valve de pulvérisation (4).

16. Kit de partie comprenant un récipient (2) avec un système de valve de pulvérisation (4) tel que défini dans l'une quelconque des revendications 1 à 10, et un écarteur (120) ou une pluralité d'écarteurs (120) ayant des ouvertures de taille différente (141), tel que défini dans l'une quelconque des revendication 1 à 10.

17. Kit de parties selon la revendication 16, comprenant en outre un timbre (200) comprenant une partie poreuse (220) avec des pores (221).
